Europäisches Patentamt

European Patent Office

Office européen des brevets

(11) Numéro de publication: **0 126 665**
**A1**

(12) # DEMANDE DE BREVET EUROPEEN

(21) Numéro de dépôt: **84400790.6**

(22) Date de dépôt: **19.04.84**

(51) Int. Cl.³: **A 61 L 2/04,** A 61 L 2/00

(30) Priorité: **21.04.83 FR 8306547**

(43) Date de publication de la demande: **28.11.84**
**Bulletin 84/48**

(84) Etats contractants désignés: **AT BE CH DE FR GB IT LI LU NL SE**

(71) Demandeur: **Prat, Jacques Eugène, Begadan, F-33340 Lesparre Medoc (FR)**

(72) Inventeur: **Prat, Jacques Eugène, Begadan, F-33340 Lesparre Medoc (FR)**

(74) Mandataire: **CABINET BONNET-THIRION, 95 Boulevard Beaumarchais, F-75003 Paris (FR)**

(54) Dispositif pour le nettoyage et la stérilisation thermique de lentilles de contact souples hydrophiles.

(57) L'invention concerne les lentilles de contact souples hydrophiles, elle vise l'entretien de celles-ci.

Le dispositif selon l'invention comprend une unité porte-lentilles (10) et une unité de commande (11). Après nettoyage par brassage et rinçage dans l'unité porte-lentilles (10), les lentilles (L) sont stérilisées dans cette même unité à l'aide de l'unité de commande (11) qui comporte un équipement adapté à mettre sous tension un élément chauffant (52) selon une cycle programmé de manière à effectuer une stérilisation selon la méthode de stérilisation connue en soi dite de TYNDALL. Aucune manipulation des lentilles n'est à effectuer entre le début de la phase nettoyage, et la fin de la phase stérilisation.

0126665

1

"Dispositif pour le nettoyage et la stérilisation thermique de lentilles de contact souples hydrophiles"

La présente invention concerne d'une manière générale les lentilles de contact souples, hydrophiles, et vise plus particulièrement l'entretien de celles-ci à savoir : le nettoyage et la stérilisation.

On sait que les lentilles de contact doivent être quotidiennement nettoyées, puis stérilisées.

Jusqu'à maintenant, après qu'elles aient été portées les lentilles de contact, souples hydrophiles, sont d'abord nettoyées puis ensuite stérilisées. Il est de pratique courante pour effectuer le nettoyage d'utiliser une solution adéquate et d'agir par frottement sur les deux faces des lentilles après quoi on effectue un rinçage de celles-ci par exemple avec du sérum physiologique, ou une autre solution analogue. Ces opérations sont effectuées en tenant manuellement les lentilles, ce qui implique de la part de l'utilisateur une attention soutenue, n'excluant pas les risques de détérioration par rayures, cassures, et autres déchirures, étant donné notamment que la tendance est de prévoir des lentilles de plus en plus minces et, partant, de plus en plus fragiles. De plus, les risques de contamination extérieurs doivent être réduits au maximum, ce qui exige une hygiène parfaite des mains et des ongles, avant chaque opération de nettoyage.

Cette technique est donc contraignante pour l'utilisateur de lentilles qui doit renouveler ces opérations chaque jour, indépendamment du coût relativement élevé des produits nécessaires au nettoyage.

Postérieurement au nettoyage, les lentilles doivent être stérilisées soit à l'aide de solutions chimiques, la décontamination étant dite dans ce cas "à froid", soit encore à l' aide d'appareils utilisant une source de chaleur, la stérilisation ainsi effectuée étant dite "à chaud".

Dans la présente demande, on ne prendra en considération que la technique de stérilisation dite "à chaud".

Il en est ainsi dans le brevet français déposé le 1er Février 1974 sous le N° 74 04041, et publié le 29 Août 1975

2

sous le N° 2 259 618, qui prévoit un aseptiseur thermique constitué par une cuve, dans laquelle s'étendent deux électrodes, et à l'intérieur de laquelle peut être engagé un tube contenant les lentilles de contact baignant dans un liquide approprié ; dans un tel aseptiseur la stérilisation est effectuée par mise à ébullition d'un liquide, en l'espèce de l'eau, introduit dans la cuve, ce liquide étant porté à ébullition par les électrodes, et la stérilisation des lentilles étant effectuée lorsque le liquide est évaporé.

Selon le brevet français N° 76 33671 déposé le 9 Novembre 1976 et publié le 2 Juin 1978 sous le N° 2 369 847 les lentilles sont placées, chacune, dans une cavité ménagée à cet effet dans un couvercle obturant une cuve, chacune des dites cavités recevant un liquide, tel qu'un sérum physiologique, tandis que la cuve reçoit un liquide destiné à être porté à ébullition par les électrodes s'étendant dans la cuve, en sorte qu'après évaporation du liquide en question, les lentilles sont stérilisées.

Suivant encore un autre mode de stérilisation "à chaud" enseigné par le brevet français déposé le 9 Juillet 1976 sous le N° 76 20984 et publié le 11 Février 1977 sous le N° 2 317 942, les lentilles sont stérilisées par air chaud sec, à l'aide d'un élément chauffant qui restitue la chaleur qu'il a préalablement emmagasinée pendant une durée de temps prédéterminée, la chaleur ainsi restituée agissant sur un support de lentilles de contact, baignant dans une solution appropriée.

Suivant encore un autre mode de stérilisation enseigné par le brevet français déposé le 1er Février 1974 sous le N° 74 04041 et publié le 29 Août 1975 sous le N° 2 259 618, les lentilles sont aseptisées par mise à ébullition d'un liquide contenu dans une cuve, la dose du liquide étant ajustée en sorte que la durée de l'ébullition corresponde au temps nécessaire à l'aseptisation, ladite cuve étant adaptée à recevoir un support de lentilles.

Avec les modes de stérilisation "à chaud" mentionnés ci-dessus on observe, de façon globale, que la stérilisation "à chaud" n'est pas dénuée d'inconvénients essentiellement dûs au fait que la température de stérilisation n'est pas, ou est

3

contrôlée de façon aléatoire. Or la stérilisation des lentilles devant être effectuée fréquemment, les stérilisations répétées selon les techniques actuelles ont une influence néfaste sur les caractéristiques originelles des lentilles concernées, (opacité, jaunissement). De plus, il a été constaté que périodiquement de telles lentilles devaient être confiées à un spécialiste en vue d'un nettoyage encore plus approfondi.

Ainsi qu'on le voit, le nettoyage, et la stérilisation de lentilles souples hydrophiles tels qu'ils sont pratiqués actuellement ne donnent pas entière satisfaction.

La présente invention vise à rendre plus aisé et plus efficace le nettoyage des lentilles en minimisant les risques de détérioration, et elle propose à cet effet un dispositif pour le nettoyage de lentilles de contact qui est de réalisation simple et d'utilisation commode, elle vise aussi, conjointement, un appareil de stérilisation thermique conduisant à des résultats surprenants.

Dispositif pour le nettoyage et la stérilisation thermique de lentilles de contact, souples hydrophiles, caractérisé en ce qu'il comprend, une première unité porte-lentilles apte à former une cuve pour un liquide et à recevoir un support pour au moins deux lentilles, ledit support étant monté amovible et à rotation dans la cuve, et une seconde unité de commande comportant un équipement complet propre à commander un élément chauffant, de manière à effectuer, à travers la première unité, une stérilisation des lentilles préalablement nettoyées contenues dans celle-ci par une succession de chauffages programmés en temps et température.

Selon une caractéristique de l'invention, la cuve présente globalement une forme cylindrique de révolution et elle est adaptée à recevoir, suivant son axe longitudinal le support de lentilles comportant avantageusement deux compartiments porte-lentilles décalés longitudinalement, les deux compartiments étant aussi décalés radialement par rapport audit axe de rotation.

4

Selon une autre caractéristique de la présente invention, les deux unités, en vue de leur assemblage temporaire, sont équipées de moyens d'assemblage avantageusement constitués par au moins un aimant permanent solidaire de l'une des deux unités adapté à coopérer avec un élément correspondant prévu à cet effet sur l'autre unité.

Un tel dispositif offre comparativement à la technique actuelle, divers avantages tant en ce qui concerne la phase nettoyage des lentilles, que la phase stérilisation "à chaud" pour mise en oeuvre de la méthode indiquée ci-dessus.

L'un des avantages principaux réside dans le fait que la manipulation des lentilles est pratiquement supprimée ; en effet, après mise en place des lentilles sur le support de lentilles et insertion de celui-ci à l'intérieur de l'unité porte-lentilles, l'utilisateur n'a plus à intervenir manuellement si ce n'est pour ôter les lentilles de leur support, postérieurement à la stérilisation, au moment de leur utilisation.

Un autre avantage tient au fait de la stérilisation effectuée selon la méthode de "TYNDALL" (plusieurs chauffages à une température donnée suivis de refroidissements).

L'utilisation de la méthode de stérilisation dite de "TYNDALL" à l'aide de l'unité de commande permet une stérilisation exemplaire, mais offre conjointement étant donné notamment la température de stérilisation peu élevée ( de l'ordre de 60°C maximum) de ne pas exercer sur les lentilles des contraintes néfastes susceptibles d'altérer leurs caractéristiques originelles. Il en résulte une prolongation notable de la durée de vie des lentilles, et une facilité sensiblement améliorée de l'entretien.

Ainsi qu'on le voit, l'unité porte-lentilles étant apte à assurer un nettoyage correct des lentilles sans avoir à manipuler celles-ci, et l'unité de commande étant, elle, apte à assurer une stérilisation correcte selon la méthode précitée, les risques actuellement encourus tant en ce que concerne la phase nettoyage -contamination extérieure, déchirures, rayures, etc...- que la phase stérilisation, -notamment opacité, jaunissement- sont supprimés.

Un autre avantage du dispositif selon l'invention réside dans le faible encombrement respectivement des unités porte-lentilles, et de commande qui peuvent ainsi être facilement transportées.

D'autres caractéristiques et avantages de l'invention ressortiront d'ailleurs de la description qui va suivre donnée à titre d'exemple en référence aux dessins annexés dans lesquels :

la figure 1 est une vue en perspective de la première unité dite porte-lentilles ;

la figure 2 montre en perspective le support de lentilles extrait de l'unité porte-lentilles les compartiments portant les lentilles étant en position d'ouverture ;

la figure 3 montre en coupe transversale un compartiment porte-lentilles ouvert suivant la ligne III-III de la figure 2 ;

la figure 4 est une vue analogue à la précédente dans laquelle le compartiment porte-lentilles est fermé et,

la figure 5 montre en élévation, l'unité porte-lentilles associé à l'unité de commande ;

la figure 6 est une vue latérale selon la flèche F de la figure 5.

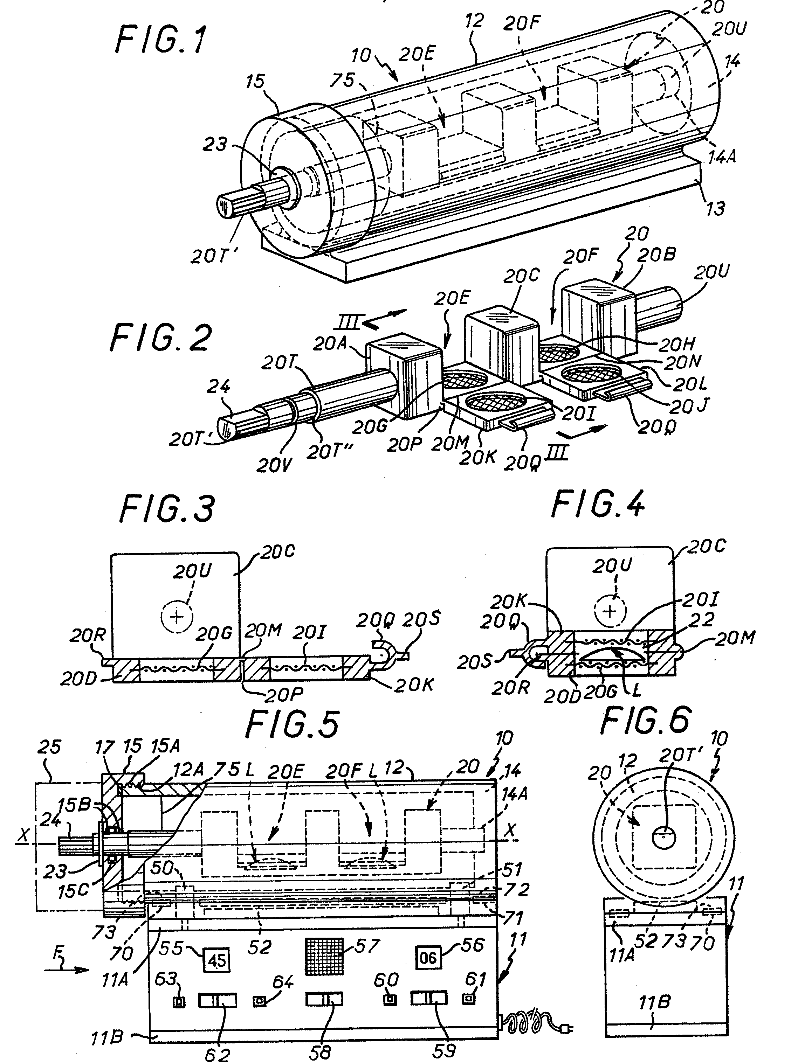

Suivant une forme de réalisation préférentielle illustrée aux figures 1 à 6, un dispositif selon l'invention comprend une unité porte-lentilles indiquée globalement en 10, et une unité de commande indiquée dans son ensemble par 11, les dites unités étant adaptées à être assemblées de manière amovible ainsi qu'on le verra plus loin.

L'unité porte-lentilles 10 est constituée d'un corps 12 globalement cylindrique avec un socle plat 13. Ce corps 12 est creux, il est obturé à une extrémité par un fond 14, et à une autre extrémité par un bouchon 15 ; le bouchon 15 peut être emboîté à l'extrémité du corps et porter un joint pour assurer l'étanchéité, mais, de préférence, et ainsi qu'il est représenté, le bouchon 15 comporte intérieurement un taraudage 15A, permettant son vissage sur un filetage 12A aménagé à l'extrémité du corps, l'étanchéité étant assurée à l'aide d'un joint circulaire 17 disposé dans le fond du bouchon.

6

Le bouchon 15 et le fond 14 du corps cylindrique 12, forment des paliers au support de lentilles indiqué globalement en 20 à la figure 2. Ce support de lentilles, avantageusement monobloc, en matière moulable, par exemple une matière plastique, est constitué par deux cloisons d'extrémité 20A, 20B espacées longitudinalement, une cloison centrale 20C, lesdites cloisons étant solidaires d'une embase 20D, de manière qu'entre les cloisons d'extrémité et centrale précitées, il est formé deux compartiments 20E, 20F. L'embase 20D forme le fond des compartiments et ceux-ci comportent, chacun dans une zone centrale, une plage ajourée 20G, 20H, avantageusement circulaire, sur lesquelles peuvent venir se superposer des plages ajourées de façon analogue 20I, 20J, ménagées dans des couvercles 20K, 20L. De préférence les couvercles précités sont venus de moulage avec le support de lentilles, et, selon la forme de réalisation représentée, les couvercles 20K, 20L, sont articulés sur l'embase au moyens de ponts 20M, 20N définis par des saignées 20P s'étendant le long de chaque compartiment, sur un côté supérieur de l'embase. Chaque bord d'extrémité des couvercles comporte d'une part, un becquet 20Q adapté à coopérer avec une languette de fermeture 20R ménagée sur l'embase 20D côté opposé à l'articulation des couvercles, et, d'autre part, une patte 20S permettant la préhension. Les deux couvercles 20K, 20L peuvent donc occuper une position ouverte, comme illustré à la figure 2 pour laquelle les lentilles peuvent être introduites, ou extraient du support de lentilles, ou une position de fermeture. La figure 4 montre le couvercle 20K dans une position de fermeture, où sa zone ajourée 20I forme avec la zone ajourée 20G ménagée dans l'embase un logement 22 dans lequel est inséré une lentille L, cette dernière étant de préférence libre dans ledit logement.

Dans le prolongement des cloisons d'extrémité 20A, 20B, le support de lentilles 20 comporte deux tronçons d'arbres cylindriques de révolution 20T, 20U, l'un, le 20T étant plus long que l'autre, comportant avantageusement une partie 20T' cannelée, ainsi qu'un méplat 24 dont l'utilité apparaîtra plus loin. Le tronçon d'arbre 20U est adapté à être emboîté dans un palier 14A ménagé à cet effet dans le fond 14, tandis que l'autre tronçon d'arbre 20T traverse un alésage 15B ménagé dans le bouchon 15, et présente une partie terminale dépassante par rapport à ce dernier, avec un épaulement 20T" destiné à limiter le jeu axial du support de lentilles à l'intérieur du corps 12 ; un joint annulaire 15C inséré dans un logement ménagé dans l'alésage précité assure l'étanchéité. Le support de lentilles 20 est avantageusement associé au bouchon 15 ; à cet effet le tronçon cylindrique 20T comporte une gorge 20V propre à l'engagement d'un circplis 23. Le support de lentilles 20 est donc associé au bouchon tout en ayant un léger jeu axial.

Le support de lentilles 20 inséré dans le corps 12 est donc susceptible de rotation autour d'un axe X-X et on observe que les lentilles L une fois emprisonnées entre les zones ajourées sont décalées radialement par rapport à cet axe X-X, en sorte qu'au cours des rotations qui peuvent être effectuées soit manuellement, soit encore à l'aide d'un moyen mécanique 25, -symbolisé en traits mixtes à la figure 5-, les lentilles L parcourent à l'intérieur du corps un trajet circulaire avec une vitesse circonférentielle permettant un effet de frottement du liquide sur les faces des lentilles.

L'unité support de lentilles, porte aussi au moins un, mais de préférence deux détecteurs de température 50, 51, qui débouchent à l'intérieur du corps cylindrique 12 et comportent des broches dépassantes par rapport à la face inférieure du socle 13 qui est aménagé pour recevoir un élément de chauffage porté par l'unité de commande.

En association avec l'unité porte-lentilles ci-dessus, l'invention prévoit une unité de commande pour une stérilisation "à chaud" des lentilles. Une telle unité de commande comprend un bloc parallélépipédique ayant un dessus 11A et un fond 11B avec sur une grande face des moyens de contrôle et de commande pour un élément chauffant.

Dans l'exemple représenté l'élément chauffant est constitué par une plaque 52 longeant en partie le dessus 11A, tandis que des moyens de contrôle tel que thermomètre 55, compte-temps 56, avertisseur sonore 57 sont prévus sur une face, ainsi que des moyens de commande tels qu'un contacteur

0126665

8

"arrêt-marche" 58 ; il est aussi prévu un contacteur 59 pour une stérilisation douce ou rapide avec témoins 60,61, et éventuellement un contacteur 62 avec témoin 63 propre à commander le moyen d'entraînement 25 en rotation du support de lentilles, dans le cas où l'unité porte-lentilles 10 est équipée d'un tel moyen mécanique. En 64 est indiqué une commande d'arrêt de l'avertisseur sonore.

Les deux unités en cause étant destinées à être associées, au moins pour la phase de stérilisation, ils comportent des moyens complémentaires pour une telle association.

Dans l'exemple représenté ces moyens sont constitués par au moins deux aimants permanents 70, 71, disposés au voisinage des extrémités de l'unité de commande et adaptés à coopérer avec des éléments métalliques correspondants 72, 73, portés par l'unité porte-lentilles, de sorte que les unités peuvent être aisément accouplées ou désaccouplées sans qu'il soit nécessaire d'avoir recours à un quelconque outillage.

L'unité de commande 11 est aménagée avec un équipement complet pour effectuer les opérations nécessaires relatives à la méthode de stérilisation dite de "TYNDALL", c'est-à-dire effectuer une stérilisation par chauffages répétés en alternance avec des périodes de refroidissement, les chauffages et refroidissements successifs étant contrôlés tant dans le temps, qu'en degrés de température. On observera ici que le placement in-situ dans la cuve de l'unité porte-lentilles, de détecteurs de température, est à même de fournir des indications remarquables de précision.

De plus, l'unité de support de lentilles 10 comporte une échelle d'indication du niveau de liquide qui peut être prévue en un quelconque endroit, par exemple comme schématisé en 75 à la figure 1, à l'intérieur du corps cylindrique et au voisinage de l'extrémité du corps côté bouchon 15.

En vue d'effectuer le nettoyage et la stérilisation de lentilles, une première phase consiste à emprisonner les lentilles à l'intérieur du support de lentilles 20 comme illustré à la figure 4 ; pour cela le bouchon 15 est dévissé, entraînant le support de lentilles 20, de sorte à l'extraire du corps 12, les couvercles 20K, 20L, sont ouverts, permet-

tant la mise en place des lentilles L dans leurs compartiments respectifs.

A cet effet, en vue d'éviter toute erreur, des repères indiquant droite, et gauche, sont prévus en un emplacement approprié par exemple sur la face supérieure de la cloison d' extrémité 20A pour lentille "gauche", et sur la face supérieure de la cloison d'extrémité 20B pour la lentille "droite" ; on referme les couvercles de sorte que les lentilles soient emprisonnées entre les zones ajourées.

On emplit ensuite l'unité porte-lentilles d'un liquide par exemple de l'eau oxygénée à 3%, ou 10 volumes; la quantité d'eau oxygénée sera avantageusement déterminée à l'aide du repère de niveau 75 prévu à l'intérieur du corps.

On met en place le support de lentilles 20 avec ses lentilles L à l'intérieur du corps 12 et on visse le bouchon 15; puis par entraînement manuel ou mécanique, à sens de rotation alterné, ou continu, le nettoyage des lentilles est effectué par brassage. A titre d'ordre de grandeur, la durée du brassage peut être comprise entre 2 et 6 minutes ponctuée par un signal sonore bref émis toutes les deux minutes, ce signal étant continu à l'expiration des 6 minutes, et fourni par l'unité de commande 11 préalablement connecté à cet effet. On évite ainsi une immersion prolongée des lentilles dans l'eau oxygénée, par oubli de la personne effectuant l'opération de nettoyage.

Cette opération étant effectuée, on vide l'unité support de lentilles, et on emplit celle-ci d'une solution de rinçage telle que du sérum physiologique, on referme et on agite de façon manuelle ou mécanique comme ci-dessus, pendant une durée de temps de l'ordre de 2 minutes, puis l'on évacue le liquide de rinçage.

La phase nettoyage étant terminée la phase stérilisation "à chaud" peut être entreprise.

Pour ce faire, on emplit le corps 12 d'un liquide propre à la stérilisation, par exemple une solution de sérum physiologique appropriée, puis on accouple l'unité porte-lentilles 10, à l'unité de commande 11 en prenant soin de placer le méplat 24 vers le haut, en sorte que les logements 22 portant

10

les lentilles L soient placés en partie inférieure du corps cylindrique 12 pour baigner dans le liquide. On met l'unité de commande 11 en ordre de marche suivant un cycle choisi, de manière à effectuer une stérilisation suivant un procédé connu en soi, dit méthode de "TYNDALL". On notera que la stérilisation peut être "douce" ou "rapide" ; la stérilisation "douce" étant effectuée à plus faible température présente l'avantage de ne pas exercer sur les lentilles des contraintes qui à la longue risqueraient d'altérer leurs caractéristiques originelles. Toutefois, le cas échéant, l'unité de commande peut effectuer une stérilisation "rapide" qui -du strict point de vue stérilisation- donne entière satisfaction.

La stérilisation "à chaud" étant terminée, l'opérateur est averti par l'avertisseur sonore 57.

On remarquera que la manipulation des lentilles pour effectuer le nettoyage et la stérilisation est réduite à l'insertion et à l'extraction des lentilles du support, d'où il résulte que les risques de détérioriation par rayures, chutes, etc. sont pratiquement éliminés.

L'unité de commande 11 est adaptée à être alimentée à partir d'une prise de courant usuelle, ou encore à partir d'une source de courant indépendante telle qu'une batterie d'accumulateurs.

Bien entendu l'invention n'est pas limitée au mode de réalisation choisi et représenté donné à titre d'exemple lequel est au contraire susceptible de modifications sans pour autant sortir du cadre de l'invention.

11
REVENDICATIONS

1. Dispositif pour le nettoyage et la stérilisation thermique de lentilles de contact, souples hydrophiles, caractérisé en ce qu'il comprend, une première unité (10) porte-lentilles apte à former une cuve (12) pour un liquide et à recevoir un support (20) pour au moins deux lentilles (L), le dit support (20) étant monté amovible et à rotation dans la cuve, et une seconde unité de commande 11 comportant un équipement complet propre à commander un élément chauffant (52), de manière à effectuer, à travers la première unité (10), une stérilisation des lentilles (L) préalablement nettoyées contenues dans celle-ci par une succession de chauffages programmés en temps et température.

2. Dispositif suivant la revendication 1, caractérisé en ce que l'unité porte-lentilles (10) est constituée d'un corps cylindrique (12) comportant un fond (14), un socle (13), ainsi qu'une extrémité ouverte obturable par un bouchon (15), le socle (13) étant adapté à être associé de manière amovible à l'unité de commande (11), l'élément chauffant (52) étant situé entre les deux unités en cause.

3. Dispositif suivant l'une quelconque des revendications précédentes, caractérisé en ce que le support de lentilles (20) est monté rotatif dans des paliers (14A, 15B) formés respectivement dans le fond (14) du corps cylindrique (12) et le bouchon (15) obturant l'extrémité ouverte de celui-ci.

4. Dispositif suivant l'une quelconque des revendications précédentes, caractérisé en ce que le bouchon (15) est vissable sur le corps (12), et il comporte un alésage avec au moins un joint d'étanchéité (15C) pour le passage d'un organe d'entraînement en rotation solidaire du support de lentilles (20), en l'espèce un tronçon cylindrique de révolution (20T).

5. Dispositif suivant l'une quelconque des revendications précédentes, caractérisé en ce que le support de lentilles (20) est associé au bouchon (15) au moyen d'un épaulement (20T") et d'un circlips (23) prévus sur le tronçon cylindrique de révolution (20T) coopérant avec le bouchon (15).

6. Dispositif suivant l'une quelconque des revendications précédentes, caractérisé en ce que le support de lentilles comporte une embase (20D) avec deux cloisons d'extrémité (20A, 20B) et une cloison centrale (20C) qui sont espacées longitudinalement, et qui ménagent entre elles deux compartiments (20E, 20F) ayant un fond ajouré (20G, 20H), lesdits compartiments étant obturables par un couvercle de fermeture (20K, 20L) adapté à ménager un logement (22) pour une lentille (L) libre dans ledit logement.

7. Dispositif suivant la revendication 6, caractérisé en ce que le couvercle de fermeture est solidaire de l'embase (20E) sur laquelle il est articulé par un pont (20M, 20N), le dit couvercle comportant une zone centrale ajourée (20I, 20T) en correspondance avec une zone ajourée (20G, 20H) ménagée dans l'embase (20E).

8. Dispositif suivant l'une quelconque des revendications précédentes, caractérisé en ce qu'à chaque compartiment (20E, 20F) est associé un couvercle (20K, 20L) articulé le long d'un bord de l'embase (20E).

9. Dispositif suivant la revendication 7, caractérisé en ce que le support de lentilles (20) est en matière moulée, chaque compartiment (20E, 20F) ayant son propre couvercle, et ceux-ci faisant partie intégrante dudit support, l'articulation desdits couvercles sur l'embase est formée, par une zone d'affaiblissement ménageant les ponts précités (20M, 20N).

10. Dispositif suivant l'une quelconque des revendications précédentes, caractérisé en ce que le fond des compartiments (20E, 20F) est radialement déporté par rapport à l'axe de rotation (X-X) du support de lentilles (20).

11. Dispositif suivant l'une quelconque des revendications précédentes, caractérisé en ce que le corps cylindrique (12) comporte des moyens visuels (75) propres à indiquer la quantité de liquide juste nécessaire admis dans le corps.

12. Dispositif suivant la revendication 11, caractérisé en ce que lesdits moyens sont constitués par une ligne circulaire (75) prévue à l'intérieur du corps (12) au voisinage de l'extrémité ouverte de celui-ci.

13. Dispositif suivant l'une quelconque des revendications précédentes, caractérisé en ce que le socle (13) est pourvu

13

d'au moins un aimant permanent (70,71) propre à coopérer avec un élément en correspondance (72,73) que comporte l'unité de commande (11).

14. Dispositif suivant l'une quelconque des revendications précédentes, caractérisé en ce que le corps cylindrique (12) porte au moins un détecteur de température (50,51) adapté à être relié à la seconde unité, lesdits détecteurs étant en saillie à l'intérieur du corps.

15. Dispositif suivant l'une quelconque des revendications précédentes, caractérisé en ce que le bouchon d'obturation (15) porte un micro-moteur (25) propre à l'entraînement en rotation continue ou alternée du support de lentilles (20).

16. Dispositif suivant l'une quelconque des revendications précédentes, caractérisé en ce que l'unité de commande (11) comporte un équipement complet pour asservir l'élément chauffant (52), selon un programme rapide ou doux.

17. Dispositif suivant l'une quelconque des revendications 1 ou 13, caractérisé en ce que l'élément chauffant (52) est porté par l'unité de commande, et est disposé de manière à être placé entre les deux unités, lorsque celles-ci sont associées, il est avantageusement constitué par une plaque chauffante (52) adaptée à la dissipation thermique d'un semiconducteur ou d'une résistance électrique.

18. Dispositif suivant l'une quelconque des revendications précédentes 1, 13 ou 14, caractérisé en ce que l'unité de commande (11) est équipée de moyens propres à asservir la plaque chauffante selon un programme déterminé, et de moyens de contrôle, lesdits moyens comportant un contacteur arrêt-marche (58), un avertisseur sonore (57) un compte-temps (56) un thermomètre (55) un contacteur pour stérilisation douce ou rapide (59), avec témoins (60,61), et le cas échéant, un contacteur (62) avec témoins (63,64) propre à la mise en marche d'un entraînement mécanique constitué par le micro-moteur (25).

19. Dispositif suivant l'une quelconque des revendications précédentes, caractérisé en ce que l'unité de commande est adaptée à être raccordée au réseau électrique, et/ou à une source de courant indépendante.

0126665

1/1.

FIG.1

FIG.2

FIG.3

FIG.4

FIG.5

FIG.6

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR-A-2 503 393  (G. LALANE)  <br><br>*  page  2,  lignes  20-34; revendications 1,4,5; figure 5 * | 1,3,4, 15,18, 19 | A 61 L   2/04<br>A 61 L   2/00 |
| A | DE-A-2 944 012  (P.M. ROCHER)  <br><br>*  page 12; page 13, lignes 1-10; pages 16,17; revendications 1-6,8 * | 1,14, 16-19 | |
| A | EP-A-0 021 828  (ACDC STERILENS LTD.)<br>*  page  5, lignes 29-32; page 7, lignes  5-29;  page  8,  lignes 30-33;  page  9;  revendications 1,8,11,21; figures 1,3,4 * | 1,16-19 | |
| A | GB-A-2 026 868  (RYDER INTERNATIONAL)<br><br>* page 1, lignes 114-130; page 2, lignes  1-11,83-130;  page  3, lignes  1-47;  revendications 1,2,4,5; figures 1-5 * | 1,2,4, 6,11, 12,14, 16-19 | DOMAINES TECHNIQUES RECHERCHES (Int. Cl. 3)<br><br>A 61 L<br>G 02 C<br>B 08 B |

Le présent rapport de recherche a été établi pour toutes les revendications

| Lieu de la recherche | Date d'achèvement de la recherche | Examinateur |
|---|---|---|
| LA HAYE | 26-07-1984 | SEIFERT H.U. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&  : membre de la même famille, document correspondant